# EUROPEAN PATENT APPLICATION

(11) **EP 4 715 834 A1**
(43) Date of publication of application: **25.03.2026**
(21) Application number: 24200957.9
(22) Date of filing: 18.09.2024
(51) Int. Cl.: G16H 20/30, G16H 20/40, G16H 30/40, G16H 40/63

(54) **ACCESSIBILITY TO PATIENT GUIDANCE AND ENTERTAINMENT IN MEDICAL PROCEDURES**

(71) Applicant: Koninklijke Philips N.V., 5656 AG Eindhoven (NL)
(72) Inventor: NAUTS, Sanne, Eindhoven (NL); SCHADEWALDT, Nicole, Eindhoven (NL); HEUVELINK, Annerieke, 5656AG Eindhoven (NL); TASAR, Ozgur, Eindhoven (NL); MASON, Jonathan David, Eindhoven (NL)
(74) Representative: Philips Intellectual Property & Standards

(57) **Abstract**

A patient accessibility adaptation system comprising an impairment determiner for determining impairment data of an impairment of a patient scheduled to undergo a medical procedure; an accessibility determiner for determining accessibility needs for the patient based on the determined presence and degree of the patient's impairment; and a system adapter for adapting a system associated with the medical procedure to address the determined accessibility needs for the patient.

## Description

### FIELD OF THE INVENTION

The present invention generally relates to a patient accessibility adaptation system, a method for adapting a system associated with a medical procedure to account for patient accessibility and a corresponding computer program product.

### BACKGROUND OF THE INVENTION

Undergoing a medical procedure, such as a medical exam, for instance a radiological exam, a (semi)-conscious surgery or treatment or other potentially uncomfortable or otherwise stressful examination or procedure can be difficult for all patients. Disabled patients, and patients with sensory or mobile impairments, may face more barriers and experience higher stress than other patients.

For instance, during a magnetic resonance imaging (MRI) scan, even relatively mild sensory impairments that are generally corrected with supportive aids (such as glasses or hearing aids) can negatively affect patient's experience. Due to the large magnetic field that is present in the MRI-room, patients typically need to take off glasses and hearing aids before entering the MRI suite, and leave other mobility aids (e.g., walking canes) behind. Having to enter a potentially stressful medical environment without one's usual aids can increase anxiety, generate feelings of lack of control, cause discomfort, and/or make it difficult for patients to follow instructions.

As such, it may be difficult for a patient undergoing an MRI scan who is normally reliant on visual aids, such as glasses, to correctly perceive breath holding instructions or see the timeline that informs the patient on upcoming events. Another issue may be that the patient may not be able to see or follow the entertainment properly, causing frustration or the tendency for the patient to move their head to try to get a proper focus distance. This results in a greater chance that a scan is not optimally performed due to motion caused by patient unrest or by the patient not correctly following instructions. In particularly severe cases a scan may need to be retaken due to unusable scan data. This is only one such example. Many more issues with other types of severity and type of disability are well known from clinical practice.

Anxiety and patients' ability to comply with instructions can be improved with systems that entertain, inform and guide patients (such as Philips Ambient Experience). Addressing this is particularly relevant for patients with sensory impairments or other disabilities. Such systems may be one of, or a combination of, lighting, screens and audio and/or such a system may be used to adjust any patient-facing content, guidance or interactive screens, such as fMRI screens, MR-compatible VR goggles, etc., and also includes any controllers thereof. Ideally, the entertainment and guidance system would be personalized to individual patients and their accessibility needs with regards to visual and auditory guidance and/or entertainment.

Staff needs to be aware of the presence and severity of such impairments. However, the patient does not always know the details of their impairment (e.g., the prescription strength of their eyeglasses) themselves or is not able to inform the staff due to reduced communication capabilities, stress or other factors. For staff, it can be therefore difficult to communicate with patients who have a sensory impairment or with patients with unmet accessibility needs. As the staff can then not anticipate on this, such situations can cause workflow disruptions or suboptimal scan results.

If patients' accessibility needs are not met, this can lead to impaired patient experience, impaired patient-staff communication, as well as reduced patient compliance. For example, people often automatically try to compensate for their impairment, for example visual impairment by squinting the eyes, but this can lead to muscle ache and discomfort. People with hearing impairment might try and turn their head so they can pick up the sound better - increasing the chance of moving in the scanner. Although patients can have all kinds of accessibility needs, there are some accessibility needs that are very common during MRI-scans, CT-scans, and other medical procedures. These are described in more detail below.

### Visual impairments

Sensory impairments are quite common within the general population. For example, the majority of adults in the US wears glasses (61%; US Vision council, 2022), as does the majority of Dutch adults and children aged 4 and up (62%; CBS, 2022). In older adults, vision aids and auditory aids are particularly common; for example, 97.3% of Dutch adults aged 75 and up require glasses/vision aids (CBS, 2022).

There are different refractory errors that are commonly corrected with glasses, including an inability to see things well if they are close by (hyperopia), an inability to see things well if they are far away (myopia), and/or issues withs seeing both things that are far away and things that are close by (astigmatism). These visual impairments are highly common in the general population; the majority of adults requires glasses or other corrective aids to see well. On top of refractive errors, there are other visual impairments that may affect vision, such as strabismus (crossed eyes), amblyopia (lazy eye), glaucoma and cataracts. Amongst older adults, almost the entire population requires visual aids, and a sizeable population also requires hearing aids.

### Hearing impairments

Hearing impairments are not as common as visual impairments, but still have a high incidence. Globally, an estimated 5% of the population suffers from disabling hearing loss; this is especially common amongst the elderly. Hearing loss can differ in type (e.g., affecting different frequency ranges) and severity. Because patients typically cannot take hearing aids into a scanner room, especially in an MR scanner room, and are typically in the scanner alone, patient-staff communication can be difficult during an (MRI) exam if patients are unable to hear well. This is exacerbated by the loud background noise produced by medical equipment, such as an MRI-scanner.

### Other disabilities

There are other disabilities or impairments (besides sensory impairments) that may affect a person's accessibility needs. Examples include mobility difficulties that require accessibility aids such as a walking cane, wheelchair, or crutches, which makes it difficult for patients to get on the patient table or move inside a scanner. Or disabilities may include an inability to lay still for long time (or at all), mental or psychiatric issues and psychopathologies, such as claustrophobia, (temporary) increased stress levels, reduced consciousness, resistance to follow instructions, and/or limited ability to process information or understand or follow instructions.

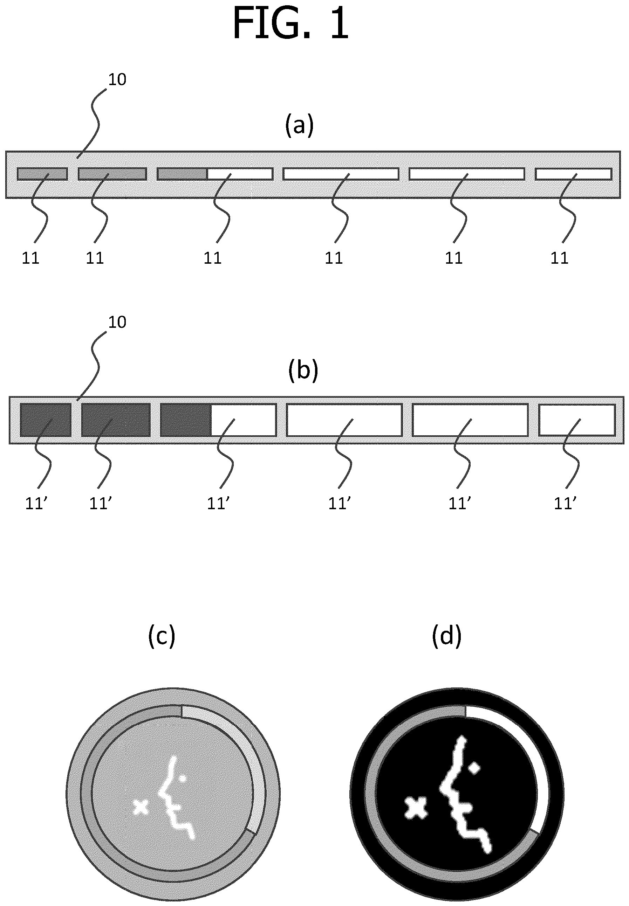

If patients have a sensory impairment or other disability, their accessibility needs may differ from the needs of people without sensory impairment or disability. For example, people with poor vision may be unable to properly see movies projected on a screen, and may be unable to see on-screen guidance, such as guidance that is currently shown to patients with regard to how long the scan will still last. An example is shown in Fig. 1a, wherein an MR scan progress bar 10 includes sequence progress bars 11 that are filled from left to right while the scan progresses, indicating to the patient when a sequence starts and ends, which usually indicate new sounds made by the scanner and the patient will be less likely to be startled. It also provides an indication to the patient how long the scan will still last to the patient, which is helpful as the patient knows how long he or she needs to stay in the same position and can have a calming effect by knowing when the scan ends. This guidance may be easy to see if a patient has good eyesight, but for a patient with a visual impairment, this guidance may be too small, or lack the required contrast between background and iconography.

Another example is guidance regarding holding one's breath, as is depicted in Fig. 1c. Here a circle slowly changing color counterclockwise over time indicates how long the patient should hold their breath. As with the previous example, the image may be too small or not have enough contrast for the patient. Chances that a breath hold instruction is not followed correctly increase because of this, potentially resulting in scans with suboptimal image quality. Having to repeat such a scan is a severe workflow problem and increases the period of discomfort for a patient, who for a next scan still may not be able to follow the breathing instructions correctly.

Thus, the accessibility need for people with poor eyesight can be to have guidance that is easily visible: e.g., icons that are large and have clear contrast. The exact type of visual impairment (e.g., the type of refractive factor and its prescription strength) can determine the exact accessibility needs. For example, a person with -3 prescription strength may require slightly larger icons (compared to a person with good eyesight), while a person with -10 prescription strength may require a different type of guidance all together (e.g., based on changes in light color, auditory instructions, etc.). Likewise, for a person with astigmatism, watching thin lines may be uncomfortable (as they can become blurry and lead to eye strain), whereas this may not be the case for a person with myopia.

While a patient with a visual impairment can have specific needs with respect to visuals, patients with auditory impairments may have a need to receive instructions in a non-auditory way. For example, instructions telling a patient that the table will move (and they need to hold still) are currently often provided in auditory form; for patients with a hearing impairment, these instructions should be provided in a different way (e.g., by showing the text on screen; by showing icons, or by showing sign language).

A patient with multiple sensory impairments (e.g., visual and auditory impairment) may require different adaptations, depending on the exact type and severity of the impairments.
For patients with mobility-related accessibility needs, proper information and lighting is important. In addition to this, patients with mobility-related accessibility needs may require additional adaptations of medical equipment; for example, the height of the patient table, or the positioning of handrails may need to be adjusted to an individual's accessibility needs.

It is not always known what the best way to present the instructions or entertainment is for each individual patient as the patient does not know or cannot properly convey the exact nature of the impairment.

Staff could manually adjust a patient-facing system to accommodate a patient's accessibility needs. However, doing so will require time, and hospital staff is often working under intense time pressure. Moreover, it may be difficult for staff to select the right settings, especially if settings need to be personalized to a patient's idiosyncratic accessibility needs. Furthermore, it can be difficult for staff to gauge the extent of a patient's visual, auditory, or other impairment. For example, many patients will not know their refractive error, and asking patients for this information may put an additional burden on patients in an already challenging, anxiety-provoking medical situation. As such, a system should preferably (semi-)automatically adapt to patients' accessibility needs, minimizing the burden on patients and staff.

Even if a patient is able to communicate sufficiently with the staff, asking patients for their specific accessibility needs still puts a burden on patients and staff. Likewise, patients with a mobility-related disability may benefit from adjustment of the settings of the imaging system or information-system, e.g., by adjusting the height of the patient support table.

It would therefore be beneficial if there was an option to automatically adapt system settings, patient guidance, patient communication and patient entertainment (e.g. using Ambient Experience) to patients' personal accessibility needs, without requiring any, (or with limited) manual action from staff or patients.

### SUMMARY OF THE INVENTION

Embodiments according to the present invention are directed to a patient accessibility adaptation system comprising an impairment determiner for determining impairment data of an impairment of a patient scheduled to undergo a medical procedure; an accessibility determiner for determining accessibility needs for the patient based on the determined presence and degree of the patient's impairment; a system adapter for adapting a system associated with the medical procedure to address the determined accessibility needs for the patient.

As such, a patient's impairment is determined automatically and the optimal measures are taken to accommodate for the impairment. Staff does not need to determine this, which saves time and eliminates the potential for human error. Patient experience is improved, the procedure has a greater chance of being successful as the patient's accessibility needs are met and, for instance, patient guidance or entertainment is more effective as patient's are likely more compliant and less stressed.

An embodiment of the present invention is directed towards the medical procedure being one or more of:
- a diagnostic procedure, such as a diagnostic imaging procedure, such as, but not limited to, a magnetic resonance (MR), computed tomography (CT), X-Ray, ultrasound, positron emission tomography (PET), and/or Single Photon Emission Computed Tomography (SPECT) imaging procedure;
- an interventional procedure, such as, but not limited to, catheterization, angioplasty, biopsy, endoscopy and/or ablation procedures;
- a surgical procedure, such as, but not limited to, conventional surgery, minimally invasive surgery, brain surgery, amputations and/or plastic surgery;
- a therapeutic procedure, such as, but not limited to, radiation therapy, chemotherapy, cryotherapy, heat therapy, electrical therapy, psychotherapy and/or high-intensity focused ultrasound (HIFU) therapy.

These medical procedures often have workflows that include extensive patient preparation, procedural length or require patient's to be well-informed, prepared or have to behave in a certain manner (e.g. staying still for an extended period of time, following specific instructions, etc.) during preparation or the procedure. Patients with accessibility needs often have more challenges to adhere to the required procedures and usually procedural steps or guidance needs to be adapted in these cases, which may cause delays or having to retake a procedure if the preparation or procedure is not performed well or is held up. The presently claimed invention improves the workflow by recognizing patient impairments and adapting workflow requirements pro-actively. This ensures that, while a procedure may still take longer than for non-impaired patients, the procedure is performed with optimal predictability and efficiency and at the same time may provide a better patient experience.

An embodiment of the present invention is directed towards the impairment determiner being configured to determine one or more of:
- a visual impairment, such as, but not limited to, myopia, hyperopia, presbyopia, astigmatism, blindness in one or both eyes, color blindness, glaucoma, cataracts, diabetic retinopathy and/or macular degeneration;
- an auditory impairment, such as, but not limited to hearing reduction, deafness, auditory processing disorder and/or tinnitus;
- a mobility impairment, such as, but not limited to fractures, joint inflammation, amputation, paralysis, reduced motor-function, reduced coordination, reduced balance, numbness, muscle weakness, shaking or other involuntary movements, inability to lie, sit or stand still, arthritis and/or osteoporosis;
- a mental impairment, such as, but not limited to, brain damage, psychiatric disorders, anxiety, stress, reduced consciousness and/or limited ability to process information or understand or follow instructions.

While this is an already large list of impairments, these are common and less common impairments that may be detected and addressed by the presently claimed invention. It is clear that a wide variety of impairments may influence the medical procedure and the patient's experience ranging from lighter to severe impairments and from physical to mental and cognitive impairments. A skilled person would understand that this list is non-limiting towards the broader scope of the invention and more impairments and means to address them could be envisioned using common knowledge.

An embodiment of the present invention is directed towards the impairment data including a presence of at least one impairment and, optionally, a severity of one or more of the at least one impairment.

Detecting or knowing a presence of an impairment or multiple impairments is essential to be able to address accessibility needs related to such an impairment. Knowing a severity of the impairment is often very useful to be able to optimize addressing the accessibility needs.

An embodiment of the present invention is directed towards the impairment determiner comprising one or more of:
- a manual input system for manually inputting impairment data by a person, such as a medical professional, the patient or a representative for the patient and/or administrative staff;
- an automatic input system for automatically determining impairment data from available patient-specific data, such as patient intake data, medical records, including results from previous procedures and/or reports by medical staff and/or statistical data based on the patient's characteristics, such as, for instance, age, gender, origins and/or race;
- a biometric sensor-based input system, including:
   - at least one biometric sensor configured to obtain biometric data of the patient;
   - logic to obtain impairment data of the patient from the obtained biometric data of the patient;
   - a camera-based input system, including:
      - at least one camera configured to obtain image data of the patient and/or patient accessories;
      - logic to obtain impairment data of the patient from the obtained image data of the patient;
      - a sensory-based input system, including:
         - at least one sensory-based system to obtain sensory data of the patient, such as a visual system, an auditory system and/or a tactile system;
         - logic to obtain impairment data of the patient from the obtained sensory data of the patient.

Impairment data may be determined in various or even multiple parallel manners. The process usually entails having a logic receiving and analyzing input from a sensor or previously obtained or otherwise available data to determine the impairment. There are options to detect impairments based on known, previously determined data or by measuring new data prior to the procedure. Having a variety of impairment determination options ensures that a very wide range of impairments may be detected. As such, it will be likely that all, or at least most relevant and up-to-date data is available to determine the accessibility needs for a patient.

An embodiment of the present invention is directed towards the impairment determiner comprising at least one camera-based input system that obtains image data of a visual impairment, such as, but not limited to, detecting glasses, detecting a strength of glasses, prosthetics, supports such as braces, a blind cane, stickers or patches on clothing or accessories signaling an impairment, a guiding person, a guide dog, observing and analyzing patient behavior, such as recording a distance between the face and instructions while the patient is reading or seeing them, measuring a length of time to read or see instructions and/or turning eyes or ears towards a visual or auditory instruction.

A related embodiment of the present invention is directed towards the impairment determiner comprising at least one camera-based input system that obtains image data of an auditory impairment, such as, but not limited to, a presence of hearing aids, a type of hearing aids, a patient using sign language and/or a reaction of a patient to an auditory instruction or signal.

Many impairments may be directly or indirectly visibly present through the presence of specific objects worn, held or otherwise present with the patient or by specific behavior associated with specific impairments. Visual detection means, such as cameras, are therefore excellent means to observe and detect visual cues associated with impairments. Multiple cameras may be present, for instance to circumvent obscured lines of views by having alternate lines of views available or by having different types of cameras, such as visual cameras combined with infrared cameras.

An embodiment of the present invention is directed towards the accessibility determiner being a logic configured to:
- receiving, from the impairment determiner, the patient's determined impairment data;
- accessing a database with accessibility needs associated with impairment data to obtain one or more impairment-specific accessibility needs associated with the patient's determined impairment data;
- outputting the one or more impairment-specific accessibility needs.

Once one or more impairments have been identified with a patient, then accessibility needs associated with these impairments must be selected. A database with known accessibility needs associated with impairments provides a convenient resource for a logic to determine which accessibility needs are relevant for the patient.

An embodiment of the present invention is directed towards the accessibility needs including one or more of:
- visual adaptations, such as, but not limited to, graphic lay-out adjustments, size adjustments, such as enlargements, simplifications, abstractions, provide braille text, provide haptic guidance, provide additional patient guidance by staff, colorizations, decolorizations, contrast adjustments, adding or expanding auditive guidance or entertainment and/or including or excluding text or images;
- auditory adaptations, such as, but not limited to, volume adjustments, addition or removal of spoken word, music and/or sound effects, changing language, use of headsets for communication and/or using noise-cancelling hardware or software;
- mobility-based adaptations, such as, but not limited to, patient support adjustments, increased or specialized patient preparation and support staff and means and/or use of restraining materials or sedation;
- mental or psychological adaptations, such as, but not limited to, introducing or adapting entertainment for the patient, increased patient guidance, sedation, selecting specialized staff, adapting a difficulty level, use of restraining materials or sedation.

Each impairment may have one or more accessibility need that needs to be addressed and each accessibility need may have one or more ways to be implemented. Some of these needs may have implementations addressing multiple types of impairments, while others may be highly specific to a single impairment. More options would be available for skilled persons to address the same or further needs.

An embodiment of the present invention is directed towards the system associated with the medical procedure including: medical systems, such as diagnostic, therapeutic or surgical devices; patient guidance systems; patient entertainment systems; medical support systems, such as patient supports, chairs; room lighting; patient preparation equipment; and/or medical software.

For many medical procedures there are various systems associated with the procedure that may be adapted to address an accessibility need. These may include systems and devices directly used during the procedure or systems and devices used prior or adjacent to the procedure.

An embodiment of the present invention is directed towards the system adapter being configured to automatically adapt or provide instructions to staff to adapt any one or more of patient guidance parameters or content, entertainment parameters or content, patient experience parameters or content, equipment, protocols associated with the medical procedure, room parameters, (specialized) staff allocation or scheduling and combinations thereof.

To address some accessibility needs it is necessary or more efficient to instruct the staff to take action to adjust or adapt equipment or provide personalized guidance. Also specialized staff may be scheduled or called if a specific accessibility need has to be addressed.

An embodiment of the present invention is directed towards the system adapter being configured to cause or adapt patient guidance to be provided to the patient that is specific to the detected impairment, for instance explaining iconography to hearing-impaired people, switching to an auditory description of relevant information for a visually impaired person or to a visual description of relevant information for an auditory impaired person, or highlighting mobility supports for mobility-impaired patients.

Patient guidance to inform and instruct the patient what is expected of the patient prior to and during the procedure is of essential importance to having efficient and effective procedures. Optimizing the patient guidance based on the patient's determined impairment ensures that the patient is informed and instructed in the optimal manner, thereby avoiding delays or fails in the medical procedure.

The claimed invention further relates to a corresponding method and computer program product.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which:
Fig. 1 shows examples of patient guidance systems: a scan progress bar for an MRI scan [(a) prior art; (b) according to the claimed invention] and a breathing indicator to indicate when and how long a patient needs to hold their breath during an imaging procedure [(c) prior art; (d) according to the claimed invention].
Fig. 2 shows an example of a room in which a patient can prepare for a procedure featuring various examples of patient guidance systems according to the claimed invention.
Fig. 3 shows a schematic example of equipment to determine strength of glasses.
Fig. 4 shows a schematic flow diagram of a method according to the claimed invention.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

### DETAILED DESRIPTION OF EMBODIMENTS

The claimed invention concerns a method to automatically adapt system settings, patient guidance, patient communication, and patient entertainment (e.g. Ambient Experience or similar systems) to patients' accessibility needs. For example, for people with impaired vision, screen settings can be adjusted to display higher contrast or larger iconography. For people with impaired hearing, guidance can be displayed through icons and/or text rather than presented through auditory instructions. By automatically adapting the settings of audiovisual systems or other patient-centric systems, these systems can be personalized and adapted to a patient's accessibility needs, without requiring any manual action from staff or patients.

The invention as claimed is illustrated and described with non-limiting examples of addressing a wide variation of accessibility needs with a system to automatically detect signs of a disability, sensory or other impairment; determine the associated accessibility needs, and adapt a patient guidance and/or instruction system, patient entertainment system (e.g., an audiovisual system) or other patient-centric system (e.g., a patient table) to those accessibility needs.

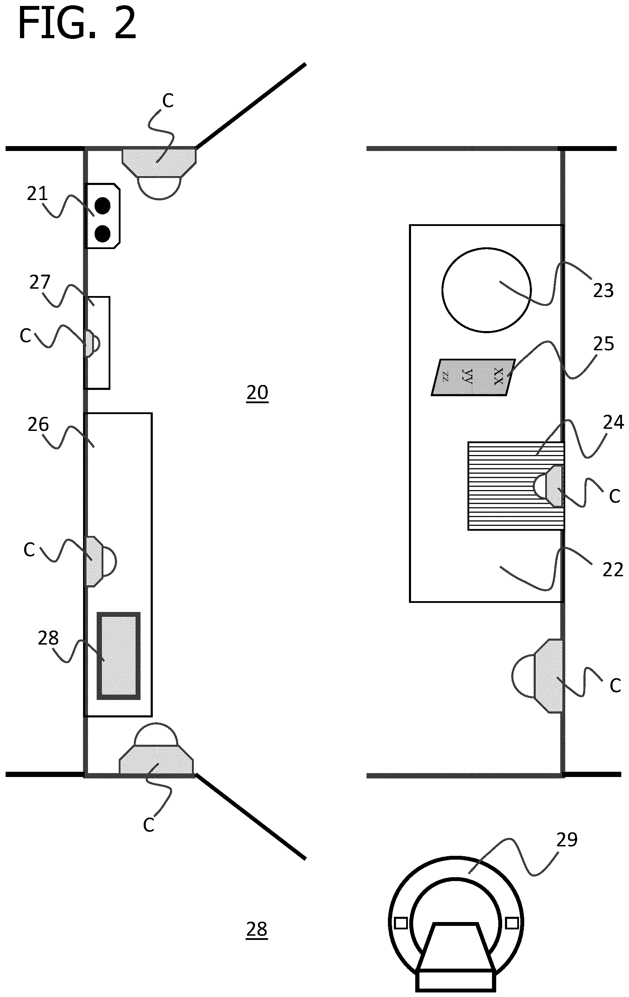

The invention is illustrated in the context of a preparation room that a patient enters prior to an examination or treatment room 20 to wait, change or otherwise prepare for the scan. Fig. 2 shows a schematic depiction of an example of such a room. The room is equipped with various embodiments of the claimed invention to detect potential impairments of a patient, and in some cases a severity thereof. Embodiments shown here may not all be present or may not be present in a single room. They may also be present in a waiting room or the procedure room, or anywhere else where the described means for impairment detection could be present. The patient may be alone in the room or accompanied by medical staff, caregiver, family member or other helper (for instance for pediatric patients or patients with severe disabilities).

The patient preparation room 20 is equipped with systems to detect signs of disability and/or (sensory) impairment, classify and quantify specific accessibility needs and trigger patient centric systems to accommodate. Some examples are given in the following non-limiting descriptions:

### Sensors, cameras and audio systems:

The preparation room 20 is equipped with one or more sensors to detect the patient's biometrics, behavior, specific items or body parts and the like. Such sensors may be cameras c such as optical cameras, 3D cameras, infra-red cameras or other specific camera systems.

The camera system can further be used to detect the patient's reaction to auditory or visual instructions and thus deduce impairments in addition to devices detected, e.g. hearing issues without hearing aids, or color blindness.

Other sensors may include audio sensors (i.e. microphones) or biometric sensors (weight, remote temperature sensors, breathing sensors, etc). Furthermore the patient may wear specific sensors that can communicate with control units in or associated with the room.

Various types of cameras c, microphones or other sensors may be positioned in different positions of the room to observe the patient and detect impairments such as hearing aids or mobility aids like crutches.

An audio system may also be installed to issue auditory instructions on the exam, e.g. on how to prepare, on where to exit the room, or similar. Or it may be used to provide background music or sounds that are pleasant to the patient.

### Visual impairment determination:

Before many exams a patient is required to remove jewelry, electronic devices, keys, glasses and the like. The preparation room 20 includes a shelf or table 22 that the patient can leave these items in a container 23 (a box, safe, dish, etc.) and hangers 21 to leave a coat or other clothes. The exemplary preparation room 20 may be further equipped with a cabinet 26 and a mirror 27. Other furniture, such as chairs, other tables, shelves and the like may be part of the room 20 too.

A camera c may detect that a patient is wearing glasses upon entry of the room or when the patient is removing them to be stored. Also, a visually impaired person may show signs in the way he/she moves through the room, for instance very carefully or close to places where the patient can hold on to something. This is an indication that visual guidance may need to be provided or entertainment may need to be adapted for this patient.

Written or other visual instructions 25 may be placed in a strategic position, for instance to inform the patient on where to store the items. A camera may detect a patient's difficulty of seeing the instructions, even if the patient is not wearing glasses. For instance, a distance the patient needs to be able to read instructions may be determined and if this is closer or further away than an average distance for a person of specific proportions, then this may indicate that that visual adaptation of patient guidance or entertainment is necessary.

If people are color blind, patient guidance and information systems may be adapted to this. Color-blindness is typically detected by asking for patterns or numbers in multi-colored circles, which are not distinguishable by color blind people. Such patterns could be displayed in the changing room with relevant information, e.g. arrows to show, where to put the glasses, or where to exit the door. These arrows could start with a color-blindness-display, but fade to a version, that is visible also for color-blind patients, and the camera can track the reaction to deduce, if the arrow (or other relevant sign) was visible to the patient before the fading.

From all such detected data it is still not exactly known how severe the visual impairment is, but this may be asked by medical staff or entered by the patient in an input device 28 present in the room 20, such as a tablet, computer or questionnaire (which may be imaged bu a camera C and automatically read and interpreted by text recognition software.

For glasses a separate, specifically adapted box 24 may be used to detect glass prescription strength detection. A sign and/or projection and/or audio guidance instructs the patient to position only glasses on this surface, and can correct the patient, if the glasses are not positioned correctly. Instead of explicit instructions, there may alternatively be a more subtle indication, e.g. the box may have the shape of a glass holder or a picture of glasses or the like. A camera c viewing this box from top is used to detect the glasses prescription strength based on distortion of an underlying pattern.

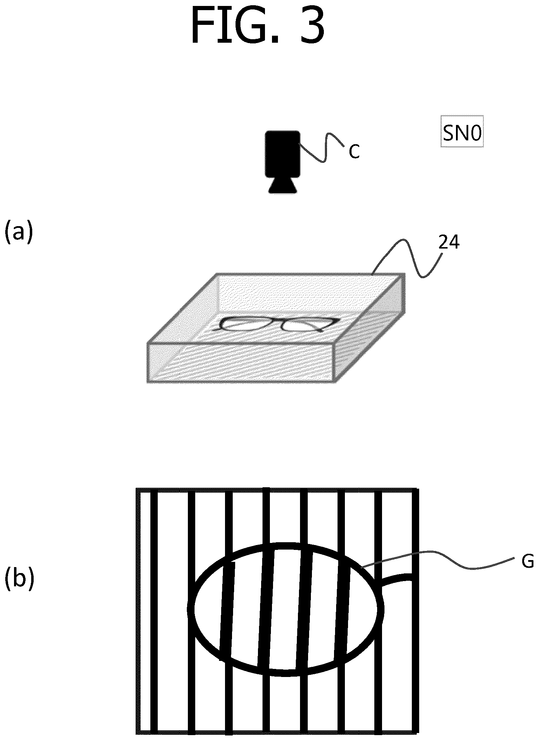

Fig. 3a illustrates an example of such a strength detection device. Glasses G are positioned on a transparent box 24 with a patterned floor (e.g. a line pattern, dotted pattern, Moiré pattern etc.). A camera c views the pattern through the glasses G and can estimate the prescription strength from the pattern distortion caused by the lens strength and shape of the glasses G. The distance between the glasses and the pattern can be fixed, or configurable, e.g. if the floor of the box 24 with the pattern is movable. The pattern may be on (fixed or interchangeable) printed media or provided by electronic means in the desired line configuration.

An example is shown in Fig. 3b. Glasses G held above a simple line pattern to display the distortion of the line pattern. Glasses on the left have, for instance, a strength of +1. In these +1 glasses, lines look thicker within the glasses than outside of it. Glasses with a negative strength will make the lines appear smaller. Glasses with a cylindrical configuration will show a slight distortion such that line thickness is different in some part of the glass than in others. As such the type and amount of distortion provides information of the specific visual impairment. A camera is used to obtain an image of the distortion, and this information is sent to a processing unit that estimates the prescription strength and type of lens of the glasses.

To ensure that such a system is maximally effective, there should be space between the patterned surface and the glasses. This may be achieved in multiple ways, e.g., by creating a thick shelf with a layer of air and a layer of transparent material (e.g. Plexiglas) between the glasses and the pattern; a transparent shelf, etc. Further, the pattern on the floor can also be created by a non-reflective screen as used for e-readers, thus the pattern and magnification of the pattern can change during the measurement for more accurate prescription strength detection.

Different patterns (Moiree patterns, etc.) or even moving images may be used that are optimally suited to detect relevant differences in prescription strength. There could be a combination of different patterns on one surface (e.g., partially striped, dotted etc.) to optimize the measurement, or a computer controllable surface to change the pattern and measure different distortions, e.g. an eReader like surface to avoid reflection.

The estimated prescription strength is sent to the patient guidance or entertainment system, to personalize the guidance or entertainment for the patient. Once a visual impairment is detected and, if possible or relevant, a severity of the impairment is known, one or more systems associated with the medical procedure may be adapted or other measures could be taken, such as for instance the estimated prescription strength can be communicated to the staff as guidance for providing correct MR-safe prescription glasses.

For instance, guidance or entertainment systems may be adapted to have enlargements, simplifications, decolorizations and/or including or excluding text or images, adding or expanding auditive instructions, or provide additional patient guidance by staff, which could include medical, support staff, volunteers or expanded involvement of people accompanying the patient to the hospital. Examples of aspects of the system that can be personalized may include:
A user interface used by patients to select entertainment options may be adapted in such a way that the choices are better visible for the patient;
Movies that patients can choose are comfortable to watch for people with poor eyesight (e.g., avoiding the use of thin lines);
For patients with severe visual impairments, light-based or sound-based guidance may be used to support them in finding their way to the position where they need to be. For example, when moving towards a door, guidance can be provided about where that door is going ("this is the door to the waiting room"; "this is the door to the MRI room"). Likewise, directional audio can be used to guide patients to the right place.

Guidance elements (such as the progress timeline showing how far along people are in the scan; breath holding guidance, etc.) are adapted to be optimally visible for patients with poor eyesight, see for instance in Figs 1b and 1d where a progress bar and a breath hold indicator have increased contrast and feature sizes compared to the standard situation (as for instance depicted in Figs 1a and 1c).

Light levels may be adapted. For instance, light levels may be increased when a patient with poor vision is in the preparation or diagnostic room. Potentially this is only done when a patient is moving or requires visual guidance. Light levels may be lowered during the procedure if that is beneficial for the outcome.

Visual guidance can be complemented by additional auditory guidance for patients with poor eyesight.

In case (near) blindness is observed, e.g. by detecting a walking cane, a guide dog, then patient interactions may be offered through tactile (e.g. braille) and/or auditory means.

Alternatively or additionally other features of the glasses may be detected to infer what the patient may need/like, e.g. detecting pink kids' glasses with a unicorn-pattern, multifocal glasses or thick-rimmed glasses. The style of the glasses suggests different types of content may be preferred by the wearer, e.g. children's content for the pink-glasses-patient; classical music and art for multifocal glasses-patient; art-related content for artistic thick-rimmed glasses.

Several more alternatives or combinations of all of these may be implemented to optimally address the specific accessibility need of a patient.

An exemplary situation involves that a patient puts her glasses on the table and the camera detects that the glasses have a prescription strength of -6. This information is used to adapt the settings of an Ambient Experience system. For example, the selection screen on which a patient can choose a theme to watch during the scan only shows themes that have been designed for people with poor eyesight; the icons and example images are displayed bigger than usual. The guidance overlays for patients (e.g., to show the timeline depicting where they are in the scan; icons to depict breath holds, etc.) are shown larger than they usually are, etc.

### Auditory impairments:

In addition to detecting glasses, camera-based measures can also be used to detect if someone is wearing hearing aids. For example, a camera c in the preparation room 20 is configured to detect if a patient has hearing aids or if they are removed during the preparation. If hearing aids are detected, the system can be set to include visual prompts (instead of or in addition to auditory instructions) to patients. These may be written prompts and/or extended iconography may be used to signal guidance or instructions to patients. Other measures may include short movie clips or sign language. If staff speaks to the patient, this text could likewise be shown on screen (as closed captions) using known text-to-speech methodologies. Also, staff or others may be deployed that know sign language.

Also, to assess a person's level of auditory impairment, patients can be exposed to different auditory cues in the waiting room to (implicitly or explicitly) asses what they can hear. An example of an implicit test is: exposing people to a sound or tone and using camera-based measures to detect if they attend to that tone (e.g., moving their head towards it).

In addition to detecting the presence of hearing aids, camera-based measures can be used to detect specific types of hearing aid a person uses, which may be indicative of the person's accessibility need and how to address it. Categorizing hearing aids can be done by comparing camera images of the hearing aid with a database of known hearing aids, or by using AI to infer the type of hearing aid.

For example, if a hearing aid is categorized as a CROS/BiCROS with a microphone on one side, the user is likely to have normal hearing/minimal hearing loss in the ear with the microphone (but impaired hearing in the other ear). Likewise, a bone conduction hearing device (e.g., a soft Baha headband) with one microphone signals that a patient suffers from hearing impairment in one specific ear. In this case, providing auditory guidance can be very helpful for patients, especially when combined with visual guidance (e.g., written text, added iconography). Other patient-centric systems may be adapted; for example, an audio system (e.g. MRI-compatible headphones), may be automatically adjusted to deliver different sound levels to both ears.

Unlike for CROS/BiCROS and single-sided bone conduction devices, other types of hearing aid may be more commonly used in patients with double-sided hearing loss. However, certain devices are recommended for patients suffering from severe hearing loss (such as behind-the-ear devices). Over-the-Counter (OTC) hearing aids are not recommended for people with severe hearing loss; for people with severe hearing loss, "power hearing aids" are recommended (from a select range of manufacturers). Thus, if a hearing aid is categorized as an Over-the-Counter model or specific type unfit for severe hearing loss, this will signal that a patient likely suffers from mild to moderate hearing loss.

Taken together, categorization of the type of hearing aid can provide information about a person's level of hearing loss, type of hearing loss, and how accessibility needs may be adapted in an optimal, personalized manner.

### Mobility impairments

When patients enter a diagnostic or treatment room (for instance an MRI-room), they frequently need to leave mobility aids (such as walking canes, wheelchairs, or crutches) behind. Using cameras or other sensors, these aids can be detected and patient-centric systems can be adjusted to optimize a patient's experience and safety.

Using cameras c in the patient preparation room 20 mobility impairments may be detected. For instance, the presence of a walking stick, a walker, prosthesis or a wheelchair may be detected. More advanced image recognition algorithms may detect mobility impairments by analysing camera data on how a patient moves through a room, for instance if a patient walks very slow or not in a straight line or needs to hold on to structures in the room or needs to stop or rest regularly. Also a patient's posture or girth may be detected, as well as a patient's height or weight. These all may be indications of mobility-related impairments that potentially need to be addressed.

Other examples of indications that a patient has a mobility impairment include: detection of stooping, posture, or specific walking patterns signaling a mobility issue; detecting walking speed, gait, signs of cerebral palsy, detecting physical features indicating a disability (e.g. missing limbs), etc. A host of other symptoms of disabilities or special needs can also be detected using camera-based measures. These include, but are not limited to, tremors, rigidity or bradykinesia (symptoms of Parkinson's), motor tics (symptoms of Tourette Syndrome/tic disorders), psychomotor agitation (symptoms of various mental disorders, dementia, and substance abuse/withdrawal), etc.

Examples of patient-centric systems that can be automatically adjusted are patient guidance systems, lighting, and patient support systems (e.g., the patient table, patient handles). For example, if mobility aids are detected, patient education content can be triggered (e.g., audio or visuals in the dressing room) to inform patients about the diagnostic or treatment room, how to navigate there and how to access it. Lighting in the diagnostic or treatment room may be adjusted to optimal brightness to help patients see where to go and how to position themselves. Patient-support systems may be adjusted to optimal height. For example, a patient support table may be automatically lowered if a wheelchair has been detected; this will help patients get onto the patient support easier and reduces the risk of falls or injuries (e.g., dislocating a shoulder when getting onto the patient support). Patient handles may be automatically raised or adjusted to match a person's accessibility need.

If camera-based measures signal that a person may have limited dexterity, staff can be informed to ask them to test if patients are able to press the nurse call. If not, additional instructions for patients may be needed to ensure that patients can signal problems to staff while in the scanner. For example, a 3D camera may spot physical compensation and coping behaviors based on impairment such as not placing pressure through the whole hand when sitting down/ standing up may indicate pain in the hands or arms. More use by one arm over another arm (or leg etc.) may imply asymmetric functioning, thus the patient may be using only one arm or leg to properly support or steady themselves. etc. The system can react with giving more time for the transfer, prompts to the staff that extra handles or body supports may be necessary, or which side to place the nurse call.

Other measures that may be taken are increased or specialized patient preparation, more or specialized support staff, increased support from present staff, and/or use of restraining materials or sedation.

When insights from detection of mobility aids (e.g., detecting a wheelchair or walking cane) are combined with sensing of the person's dimensions, this may further optimize the settings of patient-centric systems such as table height and positioning of patient handles. For example, (3D) camera-measurements of a person's height, weight, arm length etc. can be used to ensure personalized positioning of patient centric systems (such as patient handles).

If mobility-related accessibility needs are detected that can affect patient positioning, it staff may be signaled that certain patient positions are likely impossible. In this case, alternatives may be suggested (e.g., if a shoulder brace is detected, staff can be signaled that a person may be unable to assume and maintain ABER position for a shoulder MRI).

### Mental impairments

In case they are not yet known by the medical staff or if they would only occur under stressful conditions, some mental impairments may be detected prior to the medical procedure. For instance, using sensors such as cameras c or heart rate detectors may be used to detect behavior that indicates that the patient is stresses, or physiological signs of a patient becoming more stressed. A patient may start to move around the room chaotically or become catatonic. Heart rate may go up or skin color may change (e.g. reddening of the skin). Inability to listen to or follow instructions or guidance may also be an indication of a temporary or permanent mental impairment. In severe cases aggression may even be detected (e.g. patient expressing himself/herself rudely or damaging equipment). Other mental impairments may be connected to intellectual disability, which may be detected by observing a patient's ability to understand instructions. For instance, a patient may not be able to read properly or at all or he may not follow instructions due to not understanding them.

When a mental impairment is detected, and optimally linked, to a specific condition, then systems may be adapted to handle this. Instructions may be offered in different forms, guidance may be increased, more or specialized staff may be called in. In case of stress, calming music, lights or projections may be provided. In some cases the medical protocol may be adapted to be shorter or divided in sections. Furter, additional guidance or explanation may be offered or guidance may be offered in a less complex manner (e.g. less complex language, more images, less information presented at once, etc.). In extreme behavioral cases restraints or sedation may need be administered to be able to diagnose or treat the patient and this may be prepared.

### Adjusting a system to patients with multiple accessibility needs.

If a patient has multiple sensory impairments (e.g., a visual impairment and a hearing impairment, which is rather common in older adults), guidance needs to be adapted such that it is optimally accessible for patients. To this end, estimating the degree and type of visual impairment can be very beneficial. For example, not all patients with glasses will be unable to see written guidance; e.g., a patient with mild visual impairment may be able to see the guidance, while a person with a more severe visual impairment may be unable to read guidance.

### Further embodiments.

Instead of or in addition to automatically set certain parameters or equipment or personalizing Ambient Experience-settings based on detected accessibility needs, the patients' accessibility need can be communicated to staff so they can support the patient, manually adjust system settings, adjust scheduling or select correct entertainment, procedure compatible accessibility aids or guidance.

In addition to sensor-based detection of impairments, accessibility settings may also be based on existing data sources. For example, patient information (e.g., from the RIS, MRI scanner console or EMR) can be used to determine the patient's accessibility needs, or to determine a likelihood that a patient will have accessibility needs. For example, if patient data indicate that a patient is hard-of-hearing, this can be used as input for the accessibility settings. Likewise, demographic factors or other characteristics may be used to determine the likelihood that a patient has an accessibility need. For example, if a patient is aged 85 and up, the likelihood that the patient has glasses is >95%, so the default setting of the system can be that a person has visual impairment.

Logic to determine impairments may use data from sensor input or from manual or automatically provided previously available data by accessing a database with data associating determined or otherwise obtained input data with one or more specific impairments and finding the impairment(s) with associated with the input data.

When a patient's accessibility need is detected, patient education specific to the detected impairment may be automatically triggered to provide patients with information, e.g., explaining iconography to hearing-impaired people while they are in the waiting room, an auditory description of relevant information for a visually impaired person, or highlighting mobility supports for mobility-impaired patients.

As an additional indicator of accessibility need, camera-based measures of a seating position a patient selects in the waiting room can be used. For example, a person with hearing impairment may be more likely to sit in a place where they can observe staff walking in to get them. Likewise, a patient with mobility issues who needs the patient table to be high will likely select a high chair (vs. a lower chair).

In addition to visual/auditory guidance, tactile guidance may be provided in cases in which visual or auditory guidance cannot be perceived by a patient. Examples are using air puffs to signal the need to hold one's breath; tactile progress bars; and vibrating strips in the patient table that can be used to support communication.

The claimed invention is particularly useful for procedures which are relatively complex in preparation and/or execution and for which it is preferred that a patient remains at least partially conscious. Examples of such medical procedures are diagnostic imaging procedures, such as, but not limited to, magnetic resonance imaging, computed tomography, diagnostic x-ray, complex ultrasound, positron emission tomography, single-photon emission computed tomography, or therapeutic procedures such as image-guided radiation, such radiotherapy, or interventional procedures, such catheterizations, surgical procedures and other invasive procedures.

In addition to cameras or sensors placed in a preparation or dressing room, cameras or other sensors may be used with screen that patients can use to input data or select entertainment or an ambient experience theme. These are usually touch screens mounted in the preparation room or waiting room or near an entrance of the room where the procedure will take place. This screen may be equipped with cameras or other sensors (e.g., a microphone) that may be used to determine a patient's accessibility needs. Examples are:
- The camera detects that the patient is squinting while looking at the selection screen, and/or moves closely to the screen in order to be able to see the selection screen. Thus, the distance between the tablet's camera and the patient's face can be used as an indication for patient's visual acuity.
- A patient looks at the screen with glasses, and then takes off their glasses. Based on camera images of the patient's eyes with and without glasses, the approximate prescription strength (e.g., the diopter) can be calculated.
- While standing before the tablet, the patient can be prompted to indicate how their eyesight is (e.g., explicitly stating their prescription strength, saying that their eyesight is good, poor, or mediocre or by inferring this through a dedicated interaction, e.g. enlarging an image and saying 'Stop' when seen clearly or by indicating which of the offered options is readable).

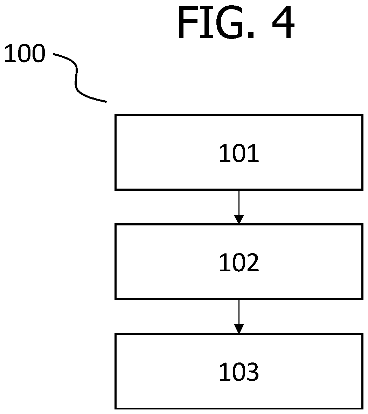

Fig. 4 shows a flowchart of the method according to the presently claimed invention, in which in step (101) impairment data is determined of an impairment of a patient scheduled to undergo the medical procedure; in step (102) accessibility needs for the patient are determined based on the determined presence and degree of the patient's impairment and in step (103) a system associated with the medical procedure is adapted to address the determined accessibility needs for the patient. This method may be implemented as a computer program product.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A patient accessibility adaptation system comprising:
- an impairment determiner for determining impairment data of an impairment of a patient scheduled to undergo a medical procedure;
- an accessibility determiner for determining accessibility needs for the patient based on the determined presence and degree of the patient's impairment;
- a system adapter for adapting a system associated with the medical procedure to address the determined accessibility needs for the patient.

2. A patient accessibility adaptation system according to claim 1, wherein the medical procedure is one or more of:
- a diagnostic procedure according to any of the previous claims, such as a diagnostic imaging procedure, such as, but not limited to, a magnetic resonance (MR), computed tomography (CT), X-Ray, ultrasound, positron emission tomography (PET), and/or Single Photon Emission Computed Tomography (SPECT) imaging procedure;
- an interventional procedure, such as, but not limited to, catheterization, angioplasty, biopsy, endoscopy and/or ablation procedures;
- a surgical procedure, such as, but not limited to, conventional surgery, minimally invasive surgery, brain surgery, amputations and/or plastic surgery;
- a therapeutic procedure, such as, but not limited to, radiation therapy, chemotherapy, cryotherapy, heat therapy, electrical therapy, psychotherapy and/or high-intensity focused ultrasound (HIFU) therapy.

3. A patient accessibility adaptation system according to any of the previous claims, wherein the impairment determiner is configured to determine one or more of:
- a visual impairment, such as, but not limited to, myopia, hyperopia, presbyopia, astigmatism, blindness in one or both eyes, color blindness, glaucoma, cataracts, diabetic retinopathy and/or macular degeneration;
- an auditory impairment, such as, but not limited to hearing reduction, deafness, auditory processing disorder and/or tinnitus;
- a mobility impairment, such as, but not limited to fractures, joint inflammation, amputation, paralysis, reduced motor-function, reduced coordination, reduced balance, numbness, muscle weakness, shaking or other involuntary movements, inability to lie, sit or stand still, arthritis and/or osteoporosis;
- a mental impairment, such as, but not limited to, brain damage, psychiatric disorders, anxiety, stress, reduced consciousness and/or limited ability to process information or understand or follow instructions.

4. A patient accessibility adaptation system according to any of the previous claims, wherein the impairment data includes a presence of at least one impairment and, optionally, a severity of one or more of the at least one impairment.

5. A patient accessibility adaptation system according to any of the previous claims, wherein the impairment determiner comprises one or more of:
- a manual input system for manually inputting impairment data by a person, such as a medical professional, the patient or a representative for the patient and/or administrative staff;
- an automatic input system for automatically determining impairment data from available patient-specific data, such as patient intake data, medical records, including results from previous procedures and/or reports by medical staff and/or statistical data based on the patient's characteristics, such as, for instance, age, gender, geographical origins and/or race;
- a biometric sensor-based input system, including:
- at least one biometric sensor configured to obtain biometric data of the patient;
- logic to obtain impairment data of the patient from the obtained biometric data of the patient;
- a camera-based input system, including:
- at least one camera configured to obtain image data of the patient and/or patient accessories;
- logic to obtain impairment data of the patient from the obtained image data of the patient;
- a sensory-based input system, including:
- at least one sensory-based system to obtain sensory data of the patient, such as a visual system, an auditory system and/or a tactile system;
- logic to obtain impairment data of the patient from the obtained sensory data of the patient.

6. A patient accessibility adaptation system according to any of the previous claims, wherein the impairment determiner comprises at least one camera-based input system that obtains image data of a visual impairment, such as, but not limited to, detecting glasses, detecting a strength of glasses, prosthetics, supports such as braces, a blind cane, stickers or patches on clothing or accessories signaling an impairment, a guiding person, a guide dog, observing and analyzing patient behavior, such as recording a distance between the face and instructions while the patient is reading or seeing them, measuring a length of time to read or see instructions and/or turning eyes or ears towards a visual or auditory instruction.

7. A patient accessibility adaptation system according to any of the previous claims, wherein the impairment determiner comprises at least one camera-based input system that obtains image data of an auditory impairment, such as, but not limited to, a presence of hearing aids, a type of hearing aids, a patient using sign language and/or a reaction of a patient to an auditory instruction or signal.

8. A patient accessibility adaptation system according to any of the previous claims, wherein the accessibility determiner is a logic configured to:
- receiving, from the impairment determiner, the patient's determined impairment data;
- accessing a database with accessibility needs associated with impairment data to obtain one or more impairment-specific accessibility needs associated with the patient's determined impairment data;
- outputting the one or more impairment-specific accessibility needs.

9. A patient accessibility adaptation system according to any of the previous claims, wherein the accessibility needs include one or more of:
- visual adaptations, such as, but not limited to, graphic lay-out adjustments, size adjustments, such as enlargements, simplifications, abstractions, provide braille text, provide haptic guidance, provide additional patient guidance by staff, colorizations, decolorizations, contrast adjustments, adding or expanding auditive guidance or entertainment and/or including or excluding text or images;
- auditory adaptations, such as, but not limited to, volume adjustments, addition or removal of spoken word, music and/or sound effects, changing language, use of headsets for communication and/or using noise-cancelling hardware or software;
- mobility-based adaptations, such as, but not limited to, patient support adjustments, increased or specialized patient preparation and support staff and means and/or use of restraining materials or sedation;
- mental or psychological adaptations, such as, but not limited to, introducing or adapting entertainment for the patient, increased patient guidance, sedation, selecting specialized staff, adapting a difficulty level, use of restraining materials or sedation.

10. A patient accessibility adaptation system according to any of the previous claims, wherein the system associated with the medical procedure includes: medical systems, such as diagnostic, therapeutic or surgical devices; patient guidance systems; patient entertainment systems; medical support systems, such as patient supports, chairs; room lighting; patient preparation equipment; and/or medical software.

11. A patient accessibility adaptation system according to any of the previous claims, wherein the system adapter is configured to automatically adapt, or provide instructions to staff to adapt any one or more of patient guidance parameters or content, entertainment parameters or content, patient experience parameters or content, equipment, protocols associated with the medical procedure, room parameters, (specialized) staff allocation or scheduling and combinations thereof.

12. A patient accessibility adaptation system according to any of the previous claims, wherein the system adapter is configured to cause or adapt patient guidance to be provided to the patient that is specific to the detected impairment, for instance explaining iconography to hearing-impaired people, switching to an auditory description of relevant information for a visually impaired person or to a visual description of relevant information for an auditory impaired person, or highlighting mobility supports for mobility-impaired patients.

13. A method for adapting a system associated with a medical procedure to account for patient accessibility comprising the steps of:
- determining (101) impairment data of an impairment of a patient scheduled to undergo the medical procedure;
- determining (102) accessibility needs for the patient based on the determined presence and degree of the patient's impairment;
- adapting (103) a system associated with the medical procedure to address the determined accessibility needs for the patient.

14. A method according to claim 1, wherein the impairment data includes a presence of at least one impairment and, optionally, a severity of one or more of the at least one impairment.

15. A computer program product configured to perform or send instructions to an executing device to perform any of the steps of claims 13 or 14.
